# EUROPEAN PATENT SPECIFICATION

(11) **EP 1 332 764 B1**
(45) Date of publication and mention of the grant of the patent: **14.12.2005**
(21) Application number: 03001453.4
(22) Date of filing: 22.01.2003
(51) Int. Cl.: A61K 47/38, A61K 47/10, A61K 47/32, A61K 31/195

(54) **A mucoadhesive thermoresponsive medicament-carrier composition**
Mucoadhäsive, wärmeaktivierbare Zubereitung als Träger für Medikamente
Composition muco-adhesive et thermosensible en tant que support de médicament

(30) Priority: 01.02.2002 CN 02103256
(43) Date of publication of application: 06.08.2003
(73) Proprietor: Pharma Power Biotec Co., Ltd., Sec. 1 Taipei (TW)
(72) Inventor: Tsui-Min, Tsai, Taipei 100, Taiwan, R.O.C. (TW)
(74) Representative: Goddar, Heinz J.

(56) References cited:
- EP-A- 0 551 626
- SHIN S-C ET AL: "Enhanced permeation of triamcinolone acetonide through the buccal mucosa" EUROPEAN JOURNAL OF PHARMACEUTICS AND BIOPHARMACEUTICS, ELSEVIER SCIENCE PUBLISHERS B.V., AMSTERDAM, NL, vol. 50, no. 2, September 2000 (2000-09), pages 217-220, XP004257193 ISSN: 0939-6411
- DESAI S D ET AL: "IN VITRO EVALUATION OF PLURONIC F127-BASED CONTROLLED-RELEASE OCULAR DELIVERY SYSTEMS FOR PILOCARPINE" JOURNAL OF PHARMACEUTICAL SCIENCES, AMERICAN PHARMACEUTICAL ASSOCIATION. WASHINGTON, US, vol. 87, no. 2, 1 February 1998 (1998-02-01), pages 226-230, XP000729422 ISSN: 0022-3549
- LIN H-R ET AL: "Carbopol/pluronic phase change solutions for ophthalmic drug delivery" JOURNAL OF CONTROLLED RELEASE, ELSEVIER SCIENCE PUBLISHERS B.V. AMSTERDAM, NL, vol. 69, no. 3, 3 December 2000 (2000-12-03), pages 379-388, XP004221288 ISSN: 0168-3659
- VONARX ET AL: "Potential efficacy of a delta 5-aminolevulinic acid bioadhesive gel formulation for the photodynamic treatment of lesions of the gastrointestinal tract in mice" JOURNAL OF PHARMACY AND PHARMACOLOGY, LONDON, GB, XP002078605 ISSN: 0022-3573

## Description

### Object of the Invention

The object of this invention is to prepare a medicament-carrier composition for the convenience of use, which has good drug delivery effect and little side effect, and which mainly comprises a mucoadhesive polymer and a thermoresponsive polymer. The medicament-carrier composition according to this invention is quite suitable for use in topical delivery of biological active compounds, especially those useful in photodynamic diagnosis or therapy, e.g. 5-aminolevulinic acid (abbreviated to "ALA").

### Background of the Invention

The final object of treating or diagnosing diseases with chemical medicaments is to deliver a suitable concentration of the drug molecules or the derivatives thereof to the site of subjects to be effected to achieve the diagnostic or therapeutic benefit while avoiding the occurrence of undesired side effect. This is especially desired for the patients afflicted with topical diseases such as skin cancer, oral cancer, tumor of larynx, leukoplakia and other mucosal diseases.

The medical effects (including toxicity and therapeutic effect) of drugs generally depend on the concentration of the action site. The most important thing is the nature of the medical active component, regardless of the systematic administration or the topical administration. For certain disease to be treated or therapentic agents, the systematic administration, could easily achieve therapeutic effects and the patient's compliance would be better. On the other hand, for the drug therapy that desires stronger effects on specific sites, the topical administration is a preferred administration route.

The administration routes for attaining topical effects generally include oral routes and topical routes. Some of the dosage forms are designed to obtain the main topical effects on the skin or the mucosa (including the skin or mucosa of eyes, nose, stomach, rectum, vagina, or airways). For example, the active charcoal adsorbent, antibacterial agents and antacids, for example, achieve topical effects in the gastrointestinal tract after oral administration.

In order to act on the specific mucosal sites successfully, the following two factors should be taken into consideration: The dosage forms should have sufficient mucoadhesive property and the retention time of the drug on the action sites should be long enough to release the drug.

The photodynamic therapy (hereinafter abbreviated to PDT) is based on the preferential adsorption and/or retention of the photosensitive chemicals by tumor tissues. The photosensitive agents are generally inert, and they will stimulate the production of toxic substances to cause the damage and death of cells, and finally kill the tumor cells when they are exposed to the radiation of certain wavelengths.

In 1990, Kennedy et al suggest the use of a photosensitive precursor "5-aminolevulinic acid (abbreviated to "ALA" for short)" in combination with PDT in the article of" Fink-Puches R, Wolf P, Kerl H et al: Photodynamic therapy of superficial basal cell carcinoma by instillation of aminolevulinic acid and irradiation with visible light. Arch Dermatol 1997; 133: 1494-1495". ALA is a protoporphyrin (protoporphyrin IX, abbreviated to PpIX, a photosensitive agent), which is a metabolic preurser of hemoglobin in the biosynthetic pathway (see A. Kübler et al., Treatment of oral leukoplakia by topical application of 5-aminolevulinic acid, *Int. J*. *Oral Maxillofac. Surg*., 1998; 27:466-469). The endogenous synthesis of ALA is modulated through the synthesis of hemoglobin by feedback control. When ALA is applied exogenously, the feedback mechanism will be surpassed and then result in overproduction and accumulation of the precursor of porphyrin , which is mostly PpIX (see Peng *et al*, "5-aminolevulinic acid-based photodynamic therapy: clinical research and future challenges", *Cancer 1997,* 79:2282-2308; Fan *et al*, "Photodynamic therapy using 5-aminolevulinie acid for premalignant and malignant lesions of oral cavity", *Cancer 1996*, 78:1374-1383; Fehr *et al*, "Selective photosensitizer localization in the human endometrium after intrauterine application of 5-aminolevulinic acid", Am J Obstet Gynecol 1996, 175:1253-1259; Svanberg *et al*, "Photodynamic therapy using intravenous delta-aminolevulinic acid-induced protoporphyrin IX sensitization in experimental hepatic tumours in rats", *Br J Cancer 1996*, 74:1526-1533).

A photodynamic reaction is initiated when PpIX is exposed to the light having certain wavelength and energy, thereby resulting in the formation of the singlet oxygen and probably peroxide and hydroxyl free radical. (see Peng *et al*, "5-aminolevulinic acid-based photodynamic therapy: clinical research and future challenges", *Cancer 1997,* 79:2282-2308; Product Information: Levulan(R) Kerastick(TM), aminolevulinic acid. DUSA Pharmaceuticals, Wilmington, MA, USA, 1999). After the exogenous application of ALA, it is found that PpIX selectively accumulates in the tumor tissue to a certain extent.

In the past ten years, numerous researches suggest that the accumulation of photosensitive agent PpIX in the tumor cells can be induced by either the systematic or topical application of ALA (see Loh CS. Vernon D. MacRobert AJ. Bedwell J. Bown SG. Brown SB. Endogenous porphyrin distribution induced by 5-aminolaevulinic acid in the tissue layers of the gastrointestinal tract. *Journal of Photochemistry & Photobiology. B.-Biology. 20*(*1*):*47*-*54*, *1993*). The therapeutic effects of ALA-mediated photodynamic therapy (abbreviated to ALA-PDT) in the precarcinomatous/carcinogenesis damage of oral and gastrointestinal tract, the transitional cell carcinoma of upper urinary tract and other conditions are disclosed. It is known that the accumulation of PpIX derived from ALA in the fast proliferating cells can be the biological theory basis for the clinical diagnosis, since PpIX will emit brownish red fluorescence under blue light having 630 nm wavelength so that the existence of tumor cells can be dignosed. (see Kriegmair *et al*., "Detection of early bladder cancer by 5-aminolevulinic acid induced prophyrin fluorescence", *J Urol 1996,* 155:105-110; Peng *et al*., "5-aminolevulinic acid-based photodynamic therapy: clinical research and future challenges", *Cancer 1997,* 79:2282-2308; Stummer *et al*., "Intraoperative detection of malignant gliomas by 5-aminolevulinic acid-induced porphyrin fluorescence", *Neurosurgery 1998*, 42:518-525). The fluorescent effect of PpIX is proven useful in the diagnosis of bladder cancer and malignant glioma.

Due to instability of ALA, there is provided a commercial product for treating skin disease employing two-chamber dosage form (Levulan®, Kerastick(TM), 1999) to obtain a better clinical effect and the convenience of operation. One of the chambers contains dried ALA power and the other contains mixed excipients. The components in the two chambers are mixed before use, the solution for topical use thus formulated should be used immediately and be discarded two hours after formulation (Prod Info Levulan® Kerastick(TM), 1999). For the safety, the mixed product solution should not contact the eyes or the surface of mucosa (Prod Info Levulan® Kerastick(TM), 1999). 14 to 18 hours after application, irradiate the administrated sites with blue light, then the photoactivation of PpIX, the metabolic product of ALA, is completed.

Apart from the application to skin, other topical applications of ALA are disclosed. For example, Michael Mehlmann et al (Lasers in Surgery and Medicine 25: 414-420, 1999) attempted to dye the tumor of larynx with fluorescence by sprayer containing 0.6 % ALA in 0.9 % NaCl solution. However, it is necessary to apply a total of 5 ml of solution containing 30 mg ALA to the patient. The volume of administration is large and the flowability of the dosage form is high. Most of ALA solution applied flows to gastrointestinal tract and is absorbed and distributed over the body, thereby resulting in undesired side effects. As it should be formulated before use and should be used immediately after formulation, it is not practical. V. Vonoxa et al (J. Pharm. Pharmacol. 1997, 49: 652-656 "Potential efficacy of a delta 5-ALA bioadhesive gel formulation for the PDT of lesions of the GI tract in mice) attempted to formulate ALA in a bioadhesive gel having uniform composition. They administrated the gel to mice to increase the conversion of ALA to PpIX. However, they agreed that: "The topical application of sensitive agents is far more suitable than systematic injection in that it is desirable to increase the concentration at the target site while decreasing the concentration in the peripheral tissue." In this research, V. Vonoxa et al attempted to design a fluid gel for oral administration to achieve the mucoadhesive effect. They concluded that 1% Noveon AA-1 (polycarbophil) gel system displays the best result, as compared with the other three systems (i.e. 2% xanthan gum, 10% carmellose sodium salt, and 15.5 % poloxamer 407).

It is found in a small research that oral administration of 60 mg/kg ALA will induce the necrosis of epithelial cells and can effectively treat the oral developmental abnormality. 12 patients suffering from leukoplakia for several years were treated by topical application of ALA in combination with PDT, 9 patients have response (but only 5 of the patients have full response. One hour before treatment, ALA was dissolved into a 20wt.% eucerin anhylic solution, then 5% arabic gum and paraffin were added to increase the adhesive uses. 3 to 5 g of the resulting mixture was applied to the affected part and covered with gauze pad. Then, 3 to 5 gm of the mixture was added once to the part every 30 minutes and said part was covered. The latex material was removed after 2 hours, then the treated part was irradiated with argon-dye laser for 1 hour. (see A. Kübler et al., Treatment of oral leukoplakia by topical application of 5-aminolevulinic acid, *Int. J. Oral Maxillofac. Surg*., 1998; 27:466-469). In this approach, every patient was administrated with a total of 1.2-2.0 gm ALA. It is believed that a substantial amount of drug would enter the blood circulation system after being swallowed and reaching the gastrointestinal tract. However, it is found that some side effects associated with the gastrointestinal tract such as nausea and vomit occured during these treatments depend on the dose of ALA (Stummer et al, "Intraoperative detection of malignant fluorescence", *Neurosurgery 1998*; 42:518-525). Thus, it is desired in this art to provide an effective topical application to reduce the occurrence of the side effects.

Poloxamers is a block copolymer having a formula HO(C₂H₄O)ₐ(C₃H₆O)_{b}(C₂H₄O)ₐH, wherein a=8-200, b=14-80, and is known as an emulsifying agent, solubilizer and wetting agent in the medical preparation. The Poloxamer is commercial available under trademark Pluronic® (BASF Corp.) or Synperonics® (ICI).

In view of the drug delivery systems for topical use disclosed in this art, the usage form, convenience and drug delivery effect are still unsatisfactory.

### Brief Summary of the Invention

This invention is related to a composition useful in photodynamic diagnosis or therapy, which comprises:
10%-30% by weight of an agent for skin or mucosa diagnosis or therapy selected from 5-aminolevulinic acid (abbreviated to "ALA") or the esterified derivatives thereof;
0.5%-2% by weight of a mucoadhesive polymer selected from the group consisting of carboxylvinylpolyester (i.e. carbopol or carbomer) and cellulose;
15%-40% by weight of a thermoresponsive polymer having two critical points, which is in solution state at a lower temperature, becomes gel state after the first critical point, and becomes solution state again at a temperature higher than the second critical point, the first critical point is between 25°C and 37°C, and the second critical point is between 45°C and 55°C;
the balance is water and/or a pharmaceutically acceptable excipient, wherein the pH value of the composition is 2-4. The medicament-carrier composition according to this invention is especially suitable for use in topical delivery of biological active compounds, especially those useful in photodynamic diagnosis or therapy, e.g. 5-aminolevulinic acid (abbreviated to "ALA") or for use in fixing the action sites of biological active compounds.

The invention is also related to a composition for photodynamic diagnosis or therapy, which mainly comprises a mucoadhesive polymer, a thermoresponsive polymer, and a photosensitive agent or the precursor thereof, e.g. 5-aminolevulinic acid (abbreviated to "ALA").

### Brief Descriptions of the Drawings

Figure 1: the elevated temperature point curve of aqueous PF-127 solution having various concentrations.
Figure 2: the reduced temperature point curve of aqueous PF-127 solution having various concentrations.
Figure 3: the effect of various concentrations of Carbopol 971P on the gel range of aqueous 25% PF-127 solution.
Figure 4: the effect of various concentrations of Carbopol 941 on the gel range of aqueous 25% PF-127 solution.
Figure 5a: the infrared analysis absorption spectrum of PF-127.
Figure 5b: the infrared analysis absorption spectrum of Carbopol 971 P.
Figure 5c: the infrared analysis absorption spectrum of the dry powder mixture of PF-127 and Carbopol 971 P in ratio 1:1.
Figure 6a: the thermograph of PF-127 which was heated at a rate of 10°C/min from 22 to 120°C and analyzed by differential scanning calorimetry (DSC). ,
Figure 6b: the thermograph of the mixture of PF-127 and Carbopol 971P of equal weight which was heated at a rate of 10°C/min from 22 to 120°C and analyzed by differential scanning calorimetry (DSC).
Figure 7a: the fluorescence spectrum of the cheek fossa of hamster after coating 10 mg of ALA in the colloidal solution system of 1% Carbopol 941 was applied.
Figure 7b: the fluorescence spectrum of the cheek fossa of hamster without the application of ALA (control side).
Figure 8a: the fluorescence spectrum of the cheek fossa of hamster after 10 mg of ALA in the collidal solution system 1% Carbopol 941 was applied.
Figure 8b: the fluorescence spectrum of the cheek fossa of hamster without the application of ALA (control side).
Figure 9a: the fluorescence spectrum of the cheek fossa of hamster after 10 mg of ALA in the colloidal solution system of 1% Carbopol 941 + 25% PF127 was applied.
Figure 9b: the fluorescence spectrum of the cheek fossa of hamster without the application of ALA (control side).
Figure 10: the fluorescence spectrum of the cheek fossa of hamster after 5 mg of ALA in different carrier compositions was applied.
Figure 11A-D: the results of clinical trials of the invention.

### The Detailed Description of the Invention

This invention is related to a composition for use in drug delivery or for use in fixing the action sites of biological active compounds, which mainly comprises a mucoadhesive polymer and a thermoresponsive polymer. The composition according to this invention is especially suitable for use in topical delivery of biological active compounds. The content of the mucoadhesive polymer in the compositon is preferably from 0.5% to 2%, the content of the thermoresponsive polymer in the composition is preferably from 15% to 40%, and the balance mainly comprises water.

The so-called "topical delivery" used herein means a dosage form applied to the epithelia of body surface to provide a topical effect at the application site. For example, a preparation is applied to the skin, cornea of eye, or mucosa of nose, rectum, vagina, or airways.

The mucoadhesive polymers suitable for the medicament-carrier composition of this invention include various known synthetic or natural polymeric materials capable of adhering to the biological mucosal surface and retaining for a period of time. The examples which can be mentioned of mucoadhesive polymers include, for example, polyacrylics such as polyacrylic acid, polyacrylates, carboxylvinyl polyesters (namely carbopol or carbomer) or a crosslinked copolymer of acrylic acid with allyl sucrose, polycarbophil or a crosslinked copolymer of acrylic acid with vinylene ethylene glycol etc.; cellulose such as carboxymethyl cellulose (CMC), carboxyethyl cellulose (HEC), hydroxypropylmethyl cellulose (HPMC), methylcellulose, chitin, etc.; natural gums such as guar gum, arabic gum, tracanth etc.; agarose and alginates etc. Carbopol (or carbomer), HPMC, HPC, CMC and guar gum are preferred, and Carbopol 971P and carbopol 941 are particularly preferred. The mucoadhesive polymers can promote the retention of drug at the mucosal sites. Thus, the combined application of drug and mucoadhesive polymers together can enhance the therapeutic effect. This administration route can be applied to the eye, nose, rectum, vagina, or airways.

The content of mucoadhesive polymers in this medicament-carrier composition is 0.5%-2%, preferably 1-1.5%, by weight of the total compositon.

The thermoresponsive polymer suitable for the medicament-carrier composition of this invention is any polymer which is in solution state at lower temperature (for example, below 25°C, preferably below 33°C), is in gel state at higher temperature (for example, about 25-60°C, preferably about 33-55°C), and is in solution state again at a much higher temperature (for example, higher than about 51°C, preferably higher than about 53°C). In other words, the thermoresponsive polymer suitable for the medicament-carrier composition of this invention has two critical points. It is in solution state at lower temperature, becomes gel state at the first critical point, and becomes solution state again at the second critical point. The first critical point is between 25°C and 37 °C, and the second critical point is between 45°C and 55°C. The examples which can be mentioned of thermoresponsive polymer especially include a block copolymer of formula HO(C₂H₄O)ₐ(C₃H₆O)_{b}(C₂H₄O)ₐH (wherein a=8-200, b=14-80), polyenamides and the like. Pluronic F-127 (abbreviated to PF-127) and poly(N-isopropylacrylamide) (abbreviated to PNIPAAM) and the like are preferred.

The content of the thermoresponsive polymer in the medicament-carrier composition of this invention is preferrably 15%-40%, particularly preferably 20-30%, by weight of the total composition.

If appropriate, the medicament-carrier composition of this invention may also comprises other pharmaceutically conventional excipients or carriers, for example, inert substances such as lactose, starch, glucose, magnesium stearate, dicalcium phosphate, mannitol, water. All the excipients can be used in combination with any known additives such as a diluent, a emulsifying agent, and a wetting agent which would not affect the adsorption and stability of active component and can be mixed by the technique well known by one skilled in medical preparation.

If appropriate, to enhance the skin penetration effect of active compounds, the medicament delivery composition of this invention may further comprises a conventional penetration enhancer capable of interfering with membrance permeability, for example, polyethylene glycol (PEG), propylene glycol(PG). In addition, of desired, the medicament delivery composition of this invention may further comprises conventional substances capable of increasing the liposolubility (or decreasing the HLB value), for example, fatty acid, to improve the liposolubility in topical administration.

The biological active compounds which can be delivered by the medicament delivery composition of this invention include any active compound which is known to be administrated via skin or mucosa of human or animal, especially those useful in photodynamic diagnosis or therapy, particularly preferably 5-aminolevulinic acid (abbreviated to "ALA"). These active compounds may be incorporated into the medicament delivery composition of this invention before use so that they are stored as a product or may be stored separately and mixed with the medicament delivery composition of this invention together in case of need.

The pH of the medicament delivery composition of this invention can be adjusted to the optimal pH by pharmaceutically conventional buffers, for example, phosphates, carbonates, acetates, etc. before or during or after mixing, depending on the active compound used. For example, if ALA is used, the pH of mixture is preferably adjusted to about 2-4. The optimal pH of various active compounds can be -adjusted by the person skilled in this art optionally.

It is found in this application surprisingly that the medicament delivery composition of this invention has the following advantages in the topical application of active compounds, especially those useful in photodynamic diagnosis or therapy (especially "ALA"):
1. It is convenient to use. Only proper application or spray is needed for administration since the drug is in liquid state at room temperature and in gel state at body temperature;
2. The retention time of the drug on the mucosal surface is relatively long without side effect;
3. The storage stability is high;
4. The dose of the active compound is low, a good effect can be attained and side effects caused by the overdose of the active compound seldom occur;
5. After taking ALA-PDT by this medicament-carrier composition, the patient is seldom photophobic and the time required for being away from strong light and sunlight may be shortened.

The effect of the medicament-carrier composition of this invention is illustrated by clinical trial using ALA. In the known art, the topical application of ALA to treat leukoplakia patients needs 30 mg of ALA and ALA should be applied several times each treatment. Clinically, at least 5 treatments should be repeated on average and the therapeutic effect therefrom is not satisfactory. For oral use, more than 60 mg/kg of ALA is needed and the side effects occur. However, when ALA is topically applied via the medicament-carrier composition of this invention, only about 10 mg of ALA is used for each treatment course and the number of treatment is reduced to at least 1/2, while a good therapeutic efficacy is attained and no side effect occurs. This outstanding medical efficacy is not suggested in any known art, and, thus, this invention substantially accomplishes the success in this aspect.

Therefore, this invention is further related to a composition useful in photodynamic diagnosis or therapy, which mainly comprises a mucoadhesive polymer, a thermoresponsive polymer and a photosensitizer or the precursor thereof, e.g. 5-aminolevulinic acid (abbreviated to "ALA"). When the medicament-carrier composition of this invention is used in combination with ALA, it is preferred to adjust the pH of the composition to less than 4 because of the high alkaline sensitivity of ALA.

In addition to be a photosensitizer, the medicament-carrier composition of this invention is quite suitable to apply an active compound which can be applied via skin or mucosa of human or animal. It can be administered easily by application or spray because it is in liquid state when applied. Moreover, it would gel spontaneously into gel state and adhers to the body surface without stripping off after contact the body temperature of a animal without stripping off, thereby facilitating an animal and human to absorb the active compound or the active compound to act directly at the affected site to develop the treatment or curing effect completely. On the other hand, it is very convenient to remove the gel from the body surface after the active compound acts for a considerable time. Thus, it is a very practical carrier composition for both medical therapy and personal care (e.g. a mask).

When the carrier composition of this invention is used for personal care (e.g. a mask), it can be further mixed with the components conventional for personal care, for example, conventional excipients, nutrients (such as vitamins, Ganoderma lucidum, collagen, etc.), humectants, etc. This application is also within the technical field of this invention.

The best mode of the representative medicament-carrier composition of this invention uses the following composition:
about 1% Carbopol 971 P;
about 20% Poloxamer PF127;
the balance of water (the percentages are based on wt./vol.).

The best application example of the representative medicament-carrier composition of this invention uses the following composition:
about 20% ALA.HCl;
about 1% Carbopol 971P;
about 20% Poloxamer PF127;
the balance of water (the percentages arc based on wt./vol.).

### Examples

For the excipient or carrier (especially excipient or carrier for medicament), the interaction between components should be avoided so that the physical property of the composition can be easily expected after the biological component is added. If a single component as such meets the criteria for ingestion or implantation, we do not desire it to harm the body as a result of the unexpected interaction with the other components. The object of this experiment is to prove that the function and basic property of each component will not change after mixing with the other components.

### Example 1.1

Pluronic F-127 (PF-127) is a polymer having revserse thermal gelation. The early reports of Achmolka (Schmolka IR. Artificial skin. I. "Preparation and properties of pluronic F-127 gels for treatment of burns", *Journal of Biomedical Materials Research. 6(6):571-82*, *1972*) and the current results of other researchers (1. Morishita M. Barichello JM. Takayama K. Chiba Y. Tokiwa S. Nagai T., "Pluronic F-127 gels incorporating highly purified unsaturated fatty acids for buccal delivery of insulin", *International Journal of Pharmaceutics. 212(2):289-93, 2001*; 2. El-Kattan AF. Asbill CS. Kim N. Michniak BB., "Effect of formulation variables on the percutaneous permeation of ketoprofen from gel formations", *Drug Delivery: Journal of Delivery & Targeting of Therapeutic Agents 7(3):147-53, 2000*; 3. Onuki Y. Morishita M. Takayama K. Tokiwa S. Chiba Y. Isowa K. Nagai T., "In vivo effects of highly purfied docosahexaenoic acid on rectal insulin absorption", *International Journal of Pharmaceutics. 198(2):147-56, 2000*) all show that at suitable aqueous concentration (suggested 20-30%), PF-127 can be liquid at lower temperature (e.g.4-5°C), become semi-solid gel-wax and almost unflowable near the body temperature, and become flowable liquid again when the temperature is increased to the second critical point.

### Experimental Process

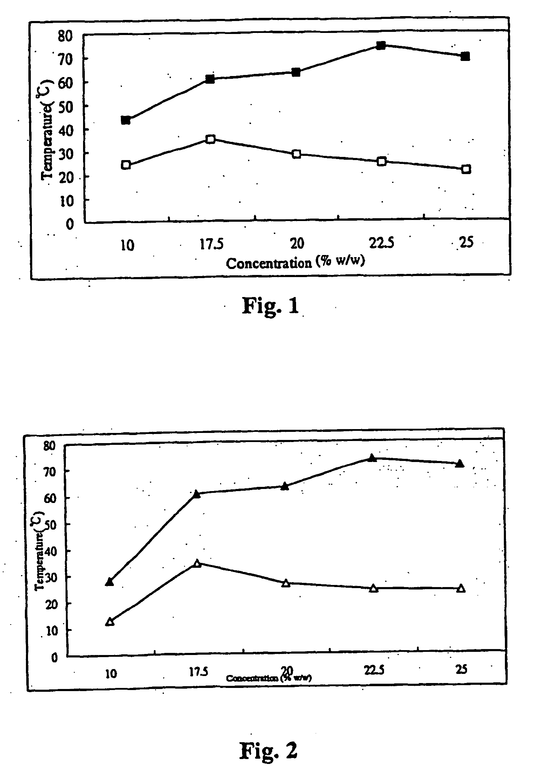

A specified amount of sample was heated from 4°C at a rate of 2°C/min at fixed volume and controlled temperature and at a speed of 400 rpm. The temperatures when the rotor stopped (T1) and started revolution again (T2) were recorded, and the operation was stopped when the temperature was increased to 90°C. The PF-127 was formulated into five concentrations of 10% w/w, 17.5% w/w, 20% w/w, 22.5% w/w,and 25% w/w. According to the procedure, the process was repeated three times to take the average, the results are shown in the Table 1 and Figure 1.

**Table 1**

| PF-127 conc. (%,w/w) | 1st critical point(°C ) | 2nd critical point(°C) |
|---|---|---|
| 10 | 24.53 | 43.6 |
| 17.5 | 35.2 | 60.5 |
| 20 | 28.27 | 62.93 |
| 22.5 | 24.87 | 73.93 |
| 25 | 21.43 | 69.13 |

### Example 1.2

The sample and operation condition are as described in example 1.1 except that the specified amount of sample was cooled from 90°C at a rate of 2°C /min at a fixed volume and controlled temperature and at a speed of 400 rpm. The temperatures at which the rotor sloped (T2) and started revolution again (T1) were recorded, and the operation was stopped when the temperature was decreased to 4°C. The PF-127 was prepared into five concentration 10% w/w, 17.5% w/w, 20% w/w, 22.5% w/w,and 25% w/w. According to the procedure, the process was repeated three times to take the average, the results are shown in the Table 2 and Figure 2.

**Table 2**

| PF-127 conc. (%,w/w) | 1^{st} critical point(°C) | 2^{nd} critical point(°C) |
|---|---|---|
| 10 | 28 | 13 |
| 17.5 | 60.47 | 34.3 |
| 20 | 62.93 | 26.33 |
| 22.5 | 73.23 | 24 |
| 25 | 70.7 | 23.67 |

### Example 1.3

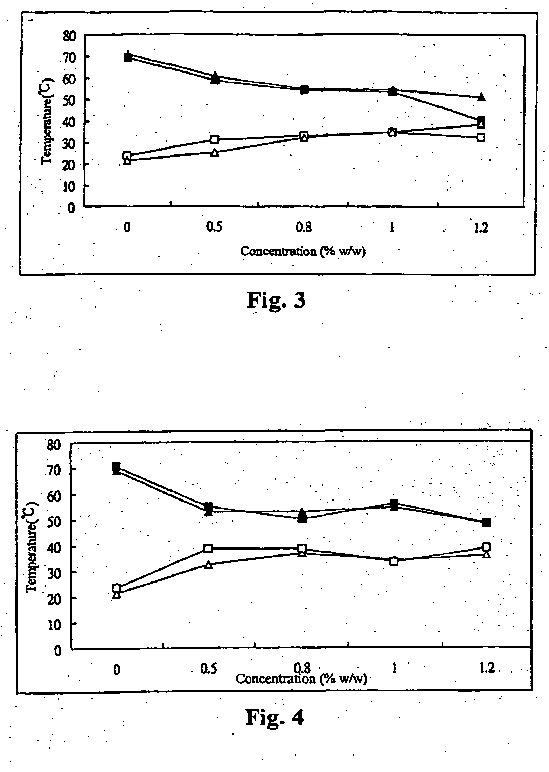

The sample and operation condition are as described in example 1.1 and example 1.2. The square represents the result of temperature increase test and triangle represents the result of temperature decrease test. The concentration of PF-127 was fixed at 25% w/w. Various concentrations of Carbopol 971P were added. Repeat the process three times and take the average. The results of the tests are shown in the Table 3 and Figure 3.

**Table 3**

| 25% w/w PF-127 + Carbopol 971 P of conc. (% w/w) | 2 ^{nd} crictical point in the temp. increase test | 2 ^{nd} crictical point in the temp. decrease test | 1 ^{st} crictical point in the temp. increase test | 1 ^{st} crictical point in the temp. decrease test |
|---|---|---|---|---|
| 0 | 70.7 | 69.13 | 23.67 | 21.43 |
| 0.5 | 60.7 | 58.67 | 31.13 | 25.17 |
| 0.8 | 54.83 | 54.33 | 33.2 | 32.37 |
| 1 | 54.2 | 53.23 | 34.7 | 34.87 |
| 1.2 | 51.37 | 40.4 | 32.73 | 38.87 |

The test results have shown that the addition of mucoadhesive polymer Carbopol 971P will not affect the first critical point and second critical point of thermoresponsive polymer PF-127 significantly. Therefore, this is an excellent carrier composition.

### Example 1.4

The sample and operation condition are as described in example 1.1 and example 1.2. The square represents the result of temperature increase test and triangle represents the result of temperature decrease test. The concentration of PF-127 was fixed at 25% w/w. Various concentrations of Carbopol 941 were added. Repeat the process three times and take the average. The results of the tests are shown in the Table 4 and Figure 4.

**Table 4**

| 25% w/w PF-127 + Carbopol 941 of conc. (% w/w) | 2 ^{nd} crictical point in the temp. increase test | 2 ^{nd} crictical the point in the temp. decrease test | 1 ^{st} crictical point in the temp. increase test | 1 ^{st} crictical point in the temp. decrease test |
|---|---|---|---|---|
| 0 | 70.7 | 69.13 | 21.43 | 23.67 |
| 0.5 | 54.87 | 52.8 | 32.43 | 38.63 |
| 0.8 | 50.17 | 52.8 | 36.83 | 38.63 |
| 1 | 55.97 | 54.53 | 34.07 | 33.37 |
| 1.2 | 48.27 | 48.43 | 36.03 | 38.93 |

The test results have shown that the addition of mucoadhesive polymer Carbopol 941 will not affect the first critical point and second critical point of thermoresponsive polymer PF-127 significantly, and thus this is an excellent carrier composition.

### Example 1.5

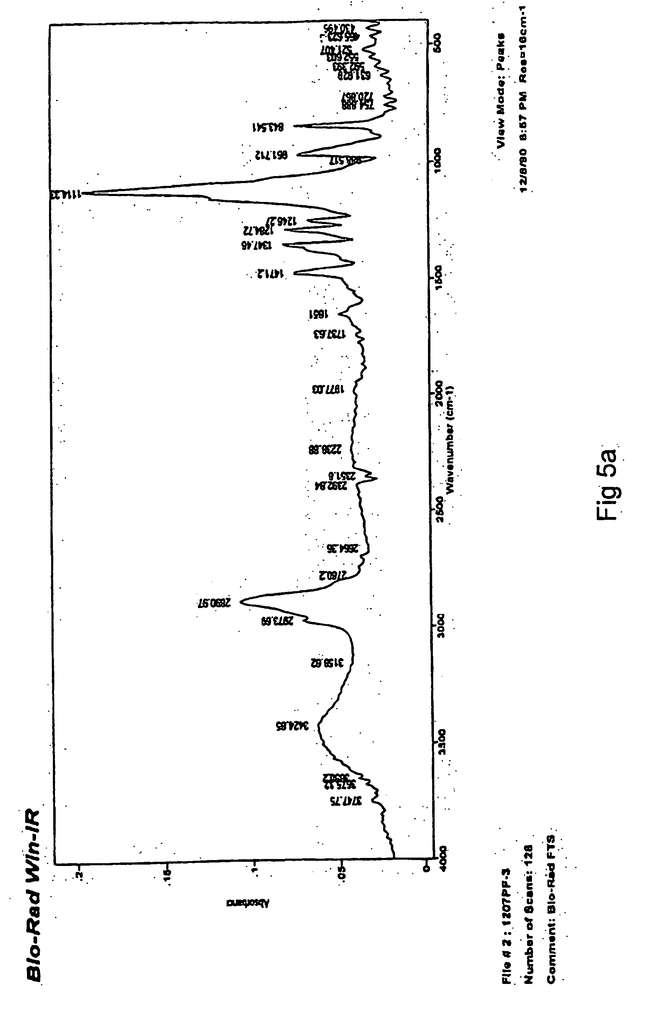

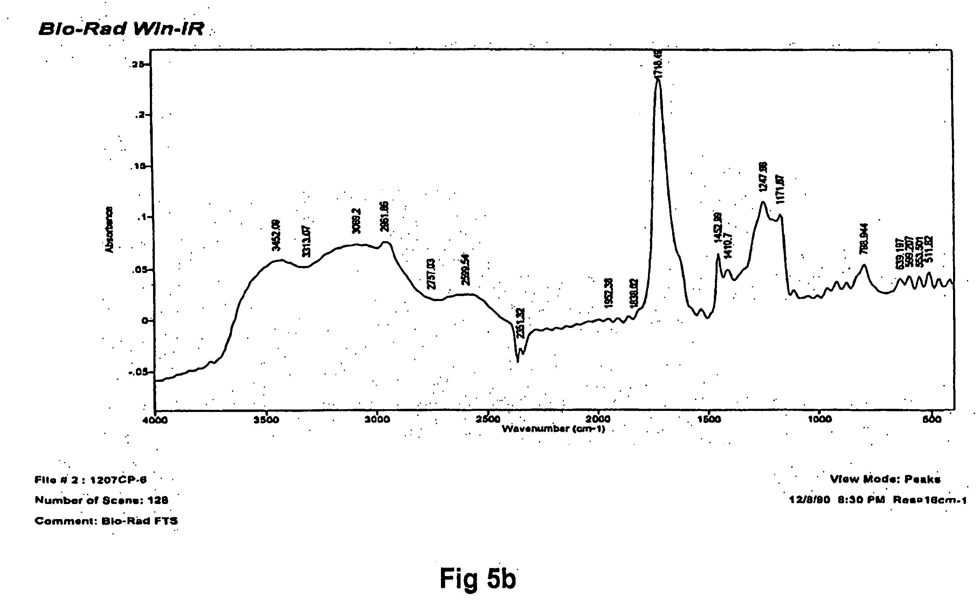

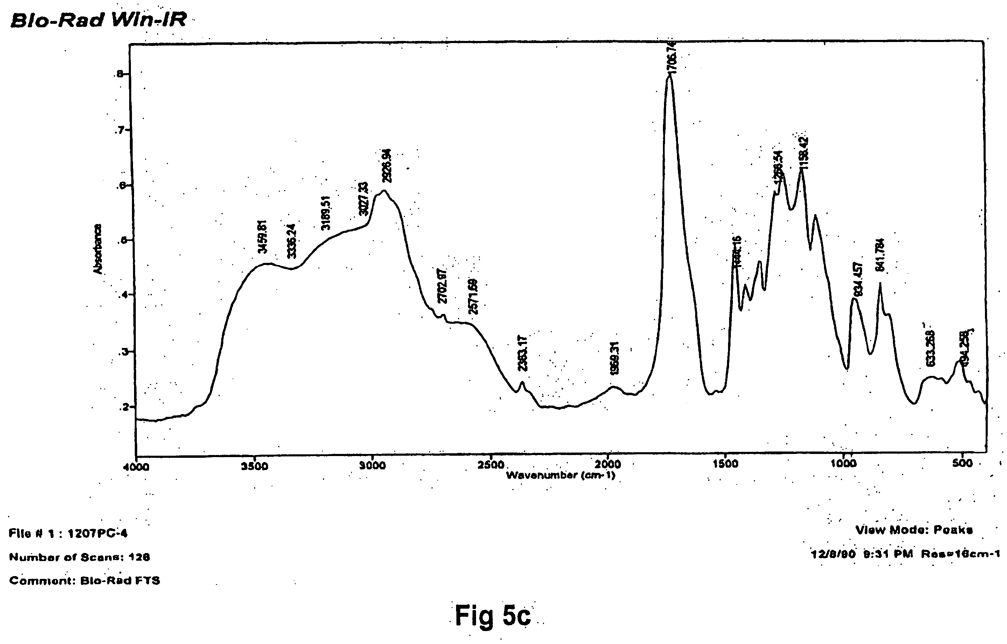

Figure 5 shows the IR analysis absorption spectrum of PF-127, Carbopol 971F, and the mixture (dry base) of PF-127 with Carbopol 971P in ratio 1:1, respectively. It is clear that no new bonding occurs between PF-127 and Carbopol 971P after mixing in solid form.

### Example 1.6

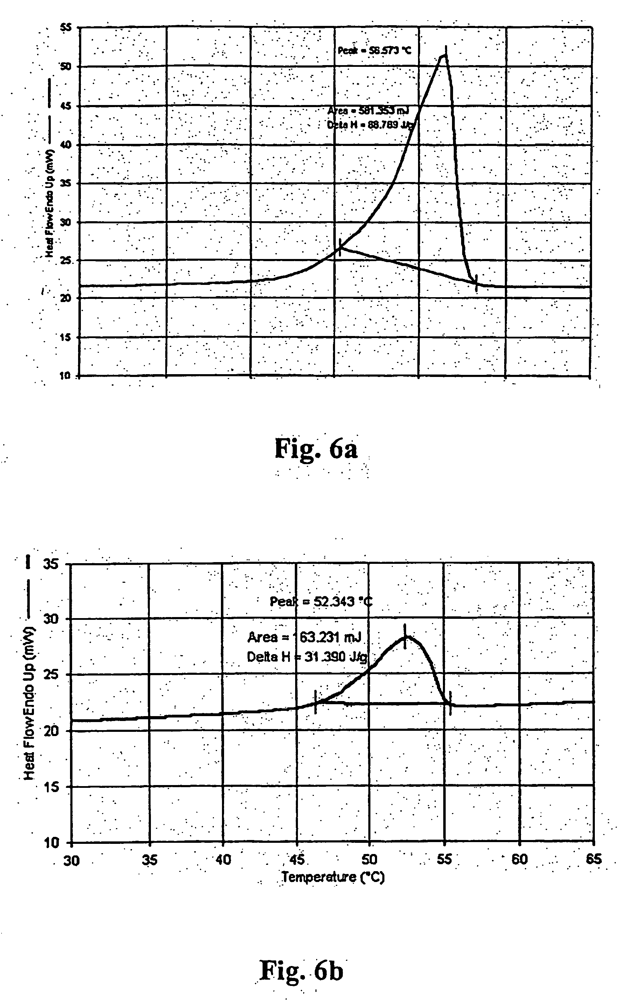

Figure 6a and 6b show the thermographs of PF-127 and Carbopol 971F heated at a rate of 10°C/min from 22 to 120°C, analyzed by differential scanning calorimetry (DSC) The thermograph shows an endothermic peak for PF-127, which represents that 56°C is the melting point thereof. There is no significant endothermic or exothermic peak between 22-120°C in the thermograph for Carbopol 971P. Although there is a slight drop in the melting point of PF-127 after the addition of Carbopol 971P, the drop is not significant and is considered to be the normal phenomenon due to the rearrangement of crystalline latice after mixing. It is recognized that the respective physico-chemical property is not affected after mixing two components because no new peak was observed.

### Pharmacological Test

### Example 2.1 P_{P}IX fluorescence test on the cheek fossa after addtion of ALA.

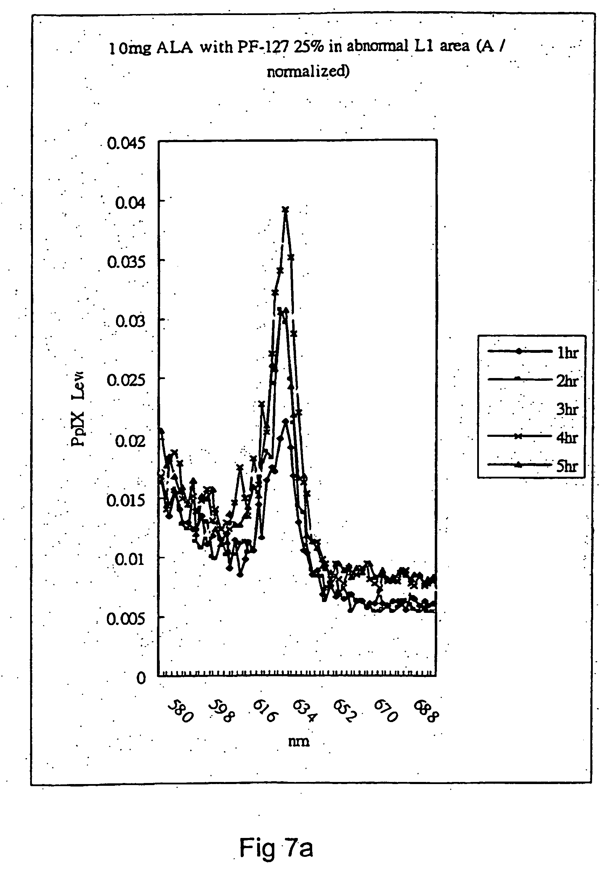

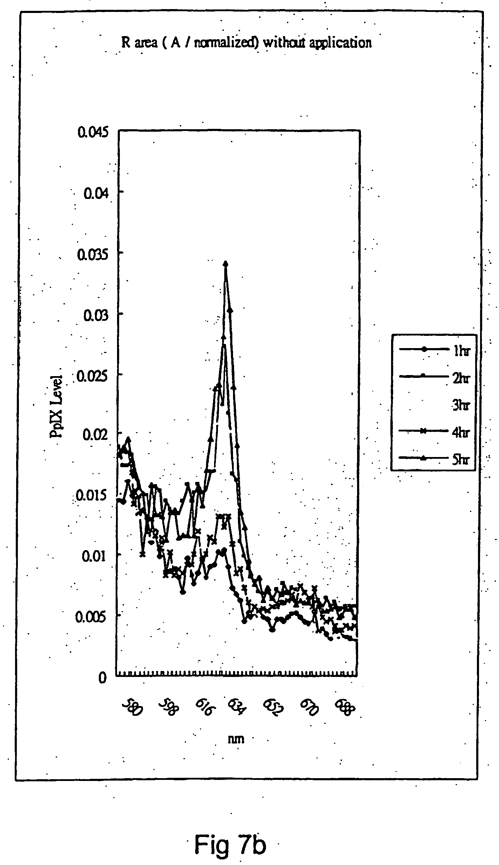

The fluorescence spectrum of the cheek fossa of hamster was obtained after 10 mg of ALA was applied. The colloidal solution system was 25% PF127 and the tissue of cheek pouch was treated with DMBA (9,10-dimethyl-1,2-benzanthracene). Figure 7a shows the side applied with ALA; Figure 7b shows the control side without the application of ALA. It can be observed from changes in fluorescence intensity that the fluorescence intensity reached the highest peak after three hours. However, the corresponding control side shows a fluorescence intensity equivalent to the application side in 5 hours. The main reason should consist in that ALA could not be controlled at the application site by PF-127 effectively.

### Example 2.2 P_{P}IX fluorescence test on the cheek fossa after addtion of ALA.

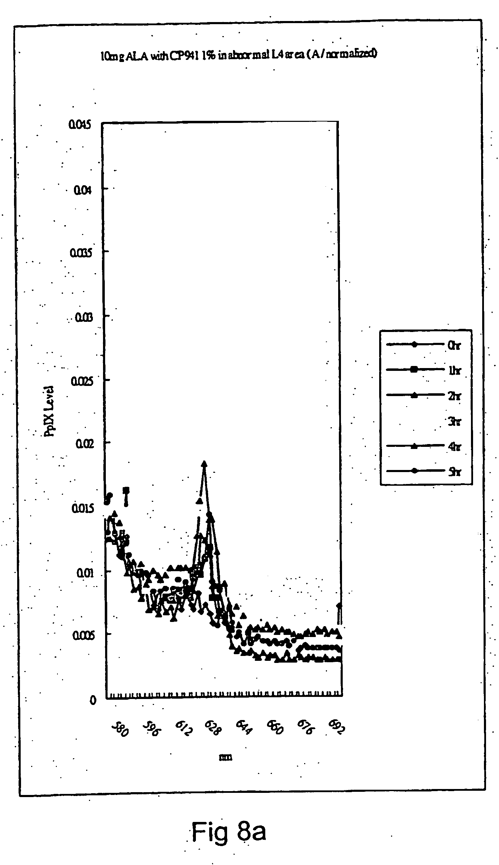

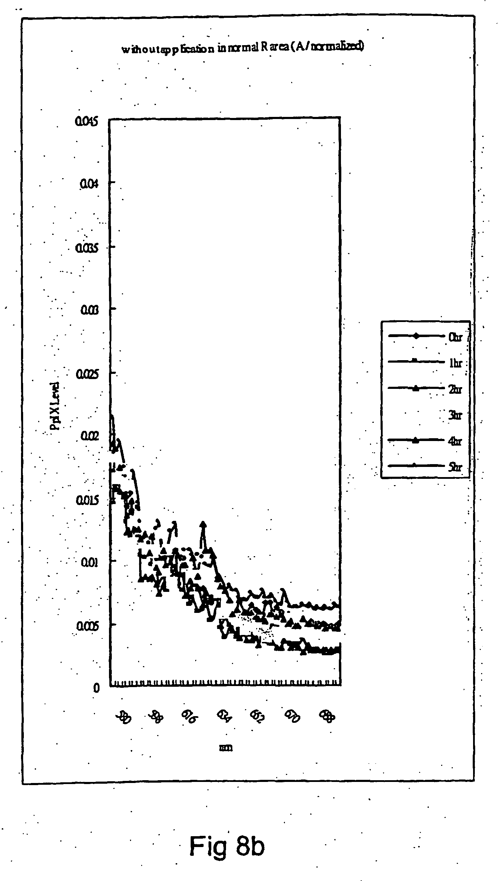

The fluorescence spectrum of the cheek fossa of hamster was obtained after 10 mg of ALA was applied. The colloidal solution system was 1% Carbopol 941 and the tissue of cheek pouch was treated with DMBA. Figure 8a shows the side applied with ALA; Figure 8b shows the control side without the application of ALA. It can be observed from the changes influorescence intensity that the fluorescence intensity reached the highest peak after 3 hours but is weaker than the fluorescence intensity of the application side of example 2.1. This phenomenon may result from the hindrance to the penetration of ALA into the tissue by the polymeric structure of Carbopol 941 (molecular weight of about one million). However, the fluorescence intensity does not show a significant change at the corresponding control side. The reason may consists in that Carbopol 941 has a better mucoadsorptive property, thereby controlling ALA to release topically.

### Example 2.3 PₚIX fluorescence test on the cheek fossa after addtion of ALA.

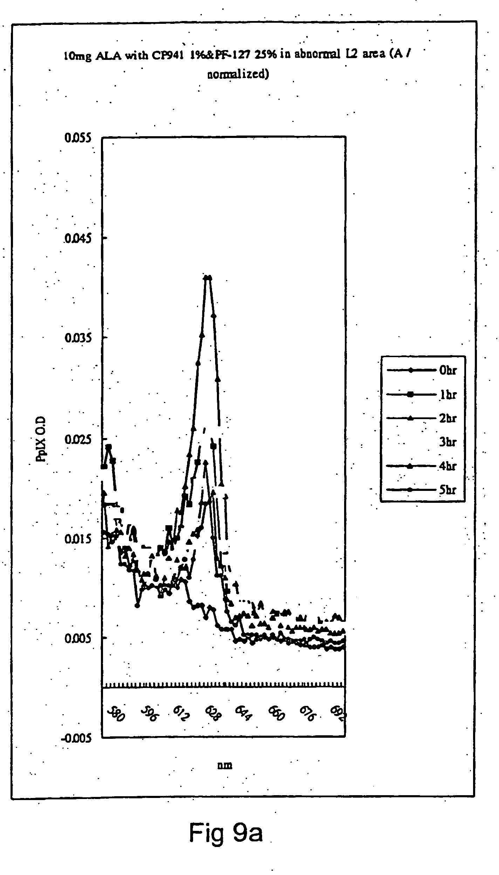

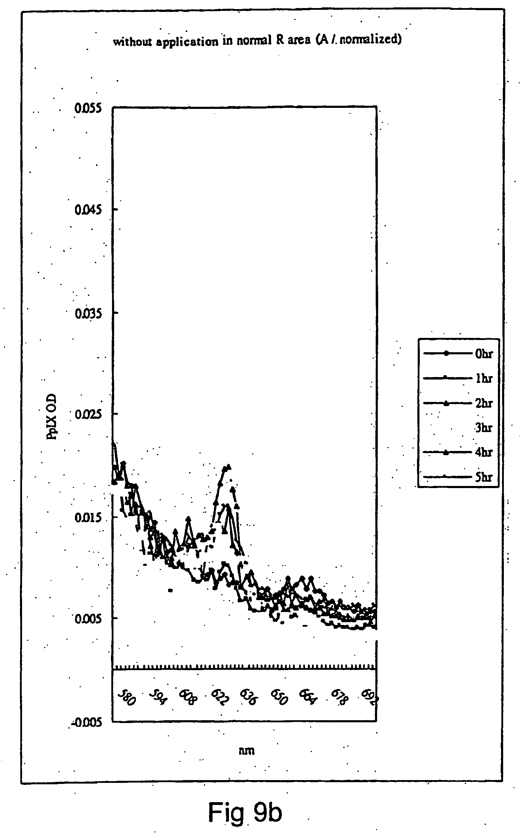

The fluorescence spectrum of the cheek fossa of hamster was obtained after 10 mg of ALA was applied. The colloidal solution system was 1% Carbopol 941 plus 25% PF-127 and the tissue of cheek pouch was treated with DMBA. Figure 9a shows the side applied with ALA; Figure 9b shows the control side without the application of ALA. It can be observed from the changes in fluorescence intensity that the fluorescence intensity reached the highest peak after two hours. The highest fluorescence intensity is equivalent to the result of example 2.1. Although there is some variation in fluorescence intensity at the corresponding control side, the variation is narrower than that in example 2.1 using PF-127 only. That could be caused by the simultaneous use of mucoadsorptive Carbopol 941 and PF-127 that may be in gel state at body temperature. Thus, this example illustrates that the original effects of the respective component mucoadhesive polymer and thermoresponsive polymer are not influenced after mixing. It shows a beneficial effect in the application.

### Example 2.4 PₚIX fluorescence test on the cheek fossa after addtion of ALA.

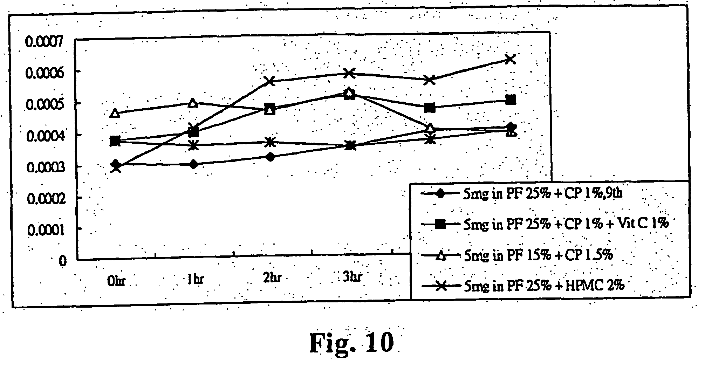

The fluorescence spectrum of the cheek fossa of hamster was obtained after 5 mg of ALA was applied employing a variety of carrier compositions. Figure 10 shows a relation plot of the average fluorescence intensity at 620-640 nm on the side applied with a variety of compositions and excited with an ercitation light of 410 nm versus time. HPMC is another mucoadsorbent, Vit.C is 1-ascorbic acid, and Fe is ferrous sulfate.

From Figure 10, it can be seen that the fluorescence intensity is weaker than that at a higher dose of ALA due to the reduced dose of ALA. As to this dose of ALA, the combination of 25% PF-127 and 2% HPMC (Methocel K100M) shows the best result. Namely, the variation in the fluorescence intensity (the difference between 0 hr and other time points) of this combination is most significant. The test result is shown in the Table 5-9 and Figure 10:

**Table 5**

| 5 mg ALA in (PF-127 25% + CP 1%), repeat 9 times | | | | | | |
|---|---|---|---|---|---|---|
| 620-640 avg | 0hr | 1hr | 2hr | 3hr | 4hr | 5hr |
| 1 | 0.0002699 | 0.000296 | 0.000399 | 0.000391 | 0.000499 | 0.000609 |
| 2 | 0.0002604 | 0.000277 | 0.000323 | 0.000348 | 0.000424 | 0.00047162 |
| 3 | 0.0002737 | 0.000281 | 0.000352 | 0.00041 | 0.000598 | 0.00042619 |
| 4 | 0.0004402 | 0.000342 | 0.000317 | 0.000339 | 0.000354 | 0.0003801 |
| 5 | 0.0003674 | 0.000298 | 0.000318 | 0.000308 | 0.000347 | 0.00037301 |
| 6 | 0.0003142 | 0.000311 | 0.000336 | 0.000346 | 0.000371 | 0.00033327 |
| 7 | 0.000283 | 0.000299 | 0.000269 | 0.000321 | 0.000305 | 0.00032559 |
| 8 | 0.0002653 | 0.000286 | 0.000265 | 0.000332 | 0.000329 | 0.00034685 |
| 9 | 0.0002598 | 0.000284 | 0.000268 | 0.000347 | 0.000336 | 0.00034764 |
| average | 0.0003038 | 0.0003 | 0.00032 | 0.00035 | 0.0004 | 0.0004015 |
| standard deviation | 61.79E-05 | 2E-05 | 4.5E-05 | 3.2E-05 | 9.6E-05 | 9.094E-05 |

**Table 6**

| 5 mg ALA in (PF-127 25% + CP 1% + Vit C 1%) | | | | | | |
|---|---|---|---|---|---|---|
| 620-640 avg | 0hr | 1hr | 2hr | 3hr | 4hr | 5hr |
| 1 | 0.00037265 | 0.000449 | 0.000514 | 0.000534 | 0.000481 | 0.0003969 |
| 2 | 0.00039621 | 0.000448 | 0.000663 | 0.000716 | 0.00057 | 0.00046648 |
| 3 | 0.00038075 | 0.000423 | 0.000623 | 0.000544 | 0.000514 | 0.00042696 |
| 4 | 0.0003987 | 0.000388 | 0.000309 | 0.000557 | 0.000602 | 0.00060228 |
| 5 | 0.00039902 | 0.000411 | 0.00051 | 0.000566 | 0.000533 | 0.00058531 |
| 6 | 0.00045477 | 0.00036 | 0.000471 | 0.000431 | 0.000336 | 0.00058604 |
| 7 | 0.00035705 | 0.000458 | 0.000381 | 0.000446 | 0.000468 | 0.00048977 |
| 8 | 0.00031595 | 0.000304 | 0.000339 | 0.000385 | 0.000356 | 0.00036271 |
| 9 | 0.00032885 | 0.000345 | 0.000431 | 0.000414 | 0.00035 | 0.00046659 |
| average | 0.0003782 | 0.0004 | 0.00047 | 0.00051 | 0.00047 | 0.000487 |
| standard deviarion | 4.159E-05 | 5.3E-05 | 0.00012 | 0.0001 | 9.8E-05 | 8.716E-05 |

**Table 7**

| 5 mg ALA in (PF-127 15% + CP 1.5%) | | | | | | |
|---|---|---|---|---|---|---|
| 620-640 avg | 0hr | 1hr | 2hr | 3hr | 4hr | 5hr |
| 1 | 0.00064041 | 0.000684 | 0.00052 | 0.000625 | 0.000483 | |
| 2 | 0.00046 | 0.000537 | 0.000468 | 0.00056 | 0.000428 | |
| 3 | 0.00047975 | 0.000552 | 0.000372 | 0.000639 | 0.000423 | |
| 4 | 0.00055916 | 0.00054 | 0.000474 | 0.00057 | 0.000419 | 0.00038254 |
| 5 | 0.0004557 | 0.000476 | 0.000428 | 0.000461 | 0.000465 | 0.0004127 |
| 6 | 0.00041084 | 0.000403 | 0.00062 | 0.000594 | 0.000372 | 0.00045964 |
| 7 | 0.00047424 | 0.000389 | 0.000462 | 0.000458 | 0.000336 | 0.00034683 |
| 8 | 0.00034183 | 0.000441 | 0.000401 | 0.000383 | 0000347 | 0.00033913 |
| 9 | 0.00036792 | 0.000409 | 0.000435 | 0.000383 | 0.000352 | 0.00037953 |
| average | 0.0004655 | 0.00049 | 0.00046 | 0.00052 | 0.0004 | 0.0003867 |
| standard deviation | 9.191E-05 | 9.6E-05 | 7.3E-05 | 0.0001 | 5.3E-05 | 4.454E-05 |

**Table 8**

| 5 mg ALA in (PF-127 25% + HPMC 2%) | | | | | | |
|---|---|---|---|---|---|---|
| 620-640 avg | 0hr | 1hr | 2hr | 3hr | 4hr | 5hr |
| 1 | 0.00027073 | 0.000371 | 0.000553 | 0.00053 | 0.000648 | 0.0008071 |
| 2 | 0.00024045 | 0.000525 | 0.000864 | 0.000618 | 0.000659 | 0.00057107 |
| 3 | 0.00026333 | 0.000646 | 0.000585 | 0.000558 | 0.000567 | 0.00058643 |
| 4 | 0.00029227 | 0.000297 | 0.000418 | 0.000366 | 0.000425 | 0.00043304 |
| 5 | 0.00029632 | 0.000357 | 0.00054 | 0.000527 | 0.000562 | 0.00058348 |
| 6 | 0.00029642 | 0.000331 | 0.000406 | 0.000583 | 0.000452 | 0.00049384 |
| 7 | 0.00028966 | 0.00031 | 0.000652 | 0.000648 | 0.000622 | 0.0074632 |
| 8 | 0.000284 | 0.000479 | 0.00059 | 0.000728 | 0.000542 | 0.00075167 |
| 9 | 0.00038285 | 0.000404 | 0.000385 | 0.000644 | 0.000513 | 0.00057985 |
| average | 0.0002907 | 0.00041 | 0.00055 | 0.00058 | 0.00055 | 0.000617 |
| standard deviation | 3.917E-05 | 0.00012 | 0.00015 | 0.0001 | 8.2E-05 | 0.0001253 |

**Table 9**

| 5 mg ALA in (PF-127 25% + CP 1% + Vit C 1% + Fe) | | | | | | |
|---|---|---|---|---|---|---|
| 620-640 avg | 0hr | 1hr | 2hr | 3hr | 4hr | 5hr |
| 1 | 0.00040509 | 0.000378 | 0.000348 | 0.000367 | 0.000456 | 0.00039485 |
| 2 | 0.00035162 | 0.000349 | 0.000402 | 0.000368 | 0.000367 | 0.00049108 |
| 3 | 0.00034912 | 0.000368 | 0.000436 | 0.000355, | 0.000354 | 0.00036006 |
| 4 | 0.00042917 | 0.000397 | 0.00037 | 0.000322 | 0.000373 | 0.00034619 |
| 5 | 0.00035238 | 0.000355 | 0.000375 | 0.000338 | 0.000355 | 0.00039839 |
| 6 | 0.00037471 | 0.000373 | 0.000323 | 0.000404 | 0.000393 | 0.00037743 |
| 7 | 0.0004043 | 0.000335 | 0.000317 | 0.000333 | 0.000339 | 0.00051095 |
| 8 | 0.00036154 | 0.000306 | 0.000347 | 0.000319 | 0.000325 | 0.00032704 |
| 9 | 0.00034233 | 0.000355 | 0.000346 | 0.000339 | 0.000343 | 0.00032848 |
| average | 0.0003745 | 0.00036 | 0.00036 | 0.00035 | 0.00037 | 0.0003927 |
| standard deviation | 3.097E-05 | 2.6E-05 | 3.8E-05 | 2.7E-05 | 3.9E-05 | 6.671E-05 |

### Clinical Trial

### Example A

A male adult took a dental examination because of oral leukoplakia. The suspected area in the mouth was applied with 0.1 ml/cm² of the composition (1% Carbopol 971 P + 20% PF-127 + 20% ALA.HCl). A film formed, coagulated and adhered to the application area without diffusion or falling off immediately, when the composition contacted the intraoral mucosa. Then wait for 1 hour to allow ALA to be absorbed by the cells at the application area. The application area was irradiated by an excitation light of 410 nm wavelength and the emission spectrum was taken from 420 nm to 700 nm at various time points (0-2 hour). After diagnosis, the composition was removed by rinsing the mouth with iced water immediately.

The fluorescence of 630 nm comes mainly from PpIX that is a metabolic product of ALA and thus shows the absorption of ALA in the composition by the tissue indirectly. The more the absorption and metabolism proceed, the intenser the fluorescence at 630 nm is. On the other hand, there are various significant differences in the absorption of ALA and the rate it metabolized into PpIX between lesion tissue and normal cells, thus the fluorescence at 630 nm can be used to identify the lesion site and the level of the lesion.

In Figure 11A, it is found that the fluorescence intensity at 630 nm during the trial tends to increase with time. The increase in fluorescence at 630 nm depends on the level of lesion. The increase in fluorescence at 630 nm of leukoplakia tissue is larger than that of normal tissue in 0-2 hr. Thus, if a suitable time point for the disease diagnosis is chosen, to the development of lesion in the mouth of the patient can be shown effectively to confirm the lesion site. And the treatment can be done at the correct site during the medical therapy.

During diagnosis process, the adhesion of said medicament-carrier composition of the invention comprising ALA to mouth is very stable. It can be seen from the fluorescence response that the absorption of ALA by the mucosal cells at the application area is very good. Therefore, this dosage form is very suitable for the diagnosis and treatment by transdermal or transmucosal administration.

### Example B

In the manner similar to example A, a male adult suffering from nasopharyngeal carcinoma went for the laryngology examination and was diagnosed by using 0.1 ml/cm² of the composition (1% Carbopol 971P + 20% PF-127 + 20% ALA.HCl). A film formed, coagulated and adhered to the application area without diffusion or falling off immediately, after the composition contacted the nasopharyngeal mucosa.

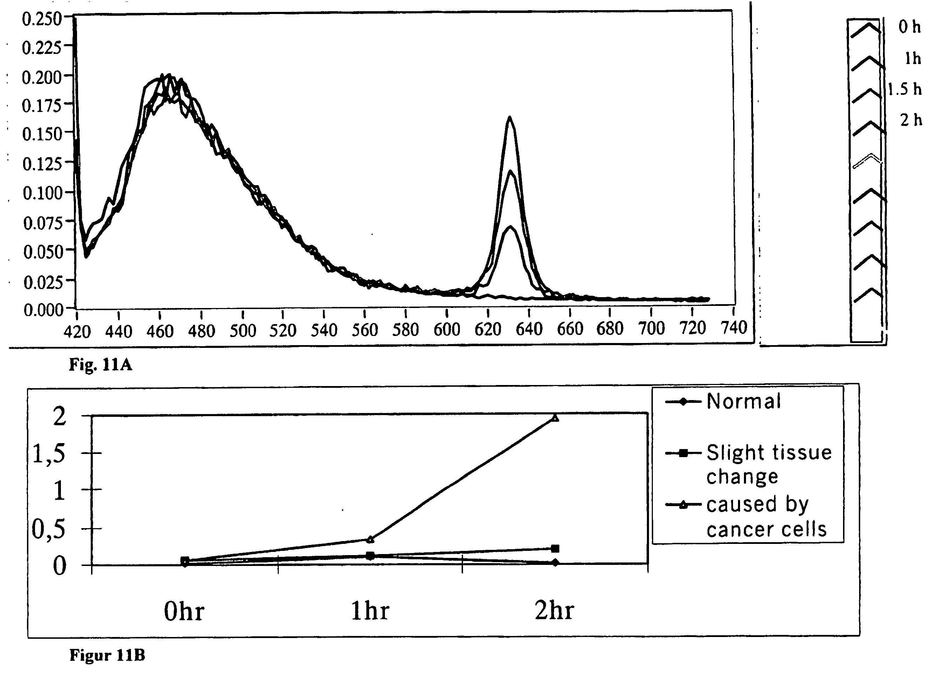

Figure 11B shows the ratio of the fluorescence intensity at 630 nm (red) to the fluorescence intensity at 460 nm (blue) (Red-Blue ratio) at different time points. In the figure, the blue, pink and yellow lines represent the measurement results of normal, mild dysplasia, and cancer sites, respectively, and the RB ratio is shown as below:

| | 0hr | 1hr | 2hr |
|---|---|---|---|
| normal | 0.016337 | 0.105675 | 0.016416 |
| mild dysplasia | 0.061702 | 0.116953 | 0.205029 |
| cancer | 0.059869 | 0.340413 | 1.936634 |

It can be seen from the Figure 11B that the fluorescence intensity at 630 nm during the trial increases with time and that the increase in fluorescence intensity at 630 nm at the cancer site is the most significant. Therefore, the distribution of cancer cells and the level of dysplasia can be diagnosed to facilitate the treatment at the correct site during the treatment course.

During diagnosis, the adhesion of the said medicament-carrier composition comprising ALA to the nasopharynx is very stable. It can be seen from the fluorescence response that the absorption of ALA by the mucosal cells at the application area is good. Therefore, this dosage form is very suitable for the diagnosis and treatment by transdermal or transmucosal administration.

### Example C

In the manner similar to example A, a male adult suffering from severe dysplasia in left cheek was diagnosed by using 0.1 ml/cm² of the composition (1% Carbopol 971P + 20% PF-127 + 20% ALA.HCl). A film formed, coagulated and and adhered to the application area without diffusion or falling off immediately, after the composition contacted the intrabuccal mucosa.

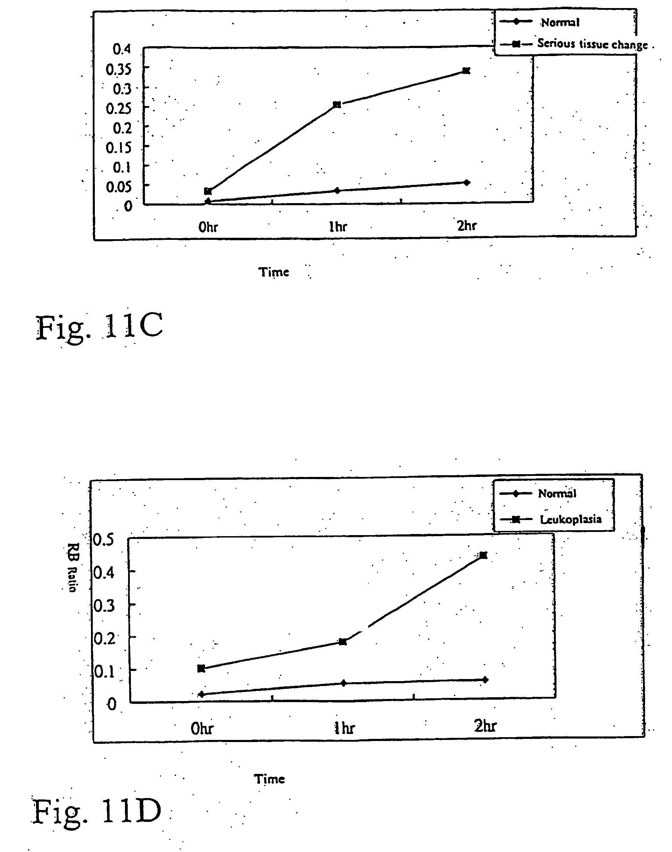

Figure 11C shows the ratio of the fluorescence intensity at 630 nm to the fluorescence intensity at 460 nm at different time points. In Figure 11C, the blue and pink lines represent the measurement results of normal and severe dysplasia, respectively, and the RB ratio is shown as below:

| | 0hr | 1hr | 2hr |
|---|---|---|---|
| normal | 0.008015 | 0.03426907 | 0.052822372 |
| severe dysplasia | 0.032451 | 0.25447475 | 0.336624826 |

It can be seen from the Figure 11C that the fluorescence intensity at 630 nm during the test increases with time and that the increase in fluorescence intensity at 630 nm at the severe dysplasia site is more prominent that at normal site. Therefore, the dysplasia site can be diagnosed, thereby facilitating the treatment at the correct site during the treatment course.

During the diagnosis, the adhesion of the said medicament-carrier composition comprising ALA to the intrabuccal part is quite stable. It can be seen from the fluorescence response that the absorption of ALA by the mucosal cells at the application area is very good. Therefore, this dosage form is quite suitable for the diagnosis and treatment by transdermal or transmucosal administration.

### Example D

In the manner similar to example A, a female adult suffering from lingual leukoplakia went for the dental examination and was diagnosed by using 0.1 ml/cm² of the composition (1% Carbopol 971P + 20% PF-127 + 20% ALA.HCl). A film formed, coagulated and adhered to the application area without diffusion or falling off immediately, after the composition contacted the lingual mucosa.

Figure 11D shows the ratio of the fluorescence intensity at 630 nm to the fluorescence intensity at 460 nm at different time points. In Figure 11D, the blue and pink lines represent the measurement results of normal and leukoplakia sites, respectively, and the RB ratio is shown as below:

| | 0hr | 1hr | 2hr |
|---|---|---|---|
| normal | 0.023081 | 0.055067 | 0.058495 |
| Leukoplakia site | 0.10061 | 0.179357 | 0.435236 |

It can be seen from the Figure 11D that the fluorescence intensity at 630 nm during the test increases with time and that the increase in fluorescence intensity at 630 nm at the leukoplakia site is more prominent than that at normal site. Therefore, the leukoplakia site can be diagnosed, thereby facilitating the treatment at the correct site during the treatment process.

During the diagnosis, the adhesion of the said medicament-carrier composition comprising ALA to the lingual part is quite stable. It can be seen from the fluorescence response that the absorption of ALA by the mucosal cells at the application area is very good. Therefore, this dosage form is quite suitable for the diagnosis and treatment by transdermal or transmucosal administration.

2 hours after the administration, the patient accepted the irradiation of the red light having wavelength 630 nm to proceed the photodynamic therapy once a week for 500 seconds per irradiation. The lingual leukoplakia almost disappeared after 4 treatments in 21 days. There is no significant undesired reaction or side effect occurring in the patient during the treatment.

## Claims

1. A composition useful in photodynamic diagnosis or therapy, which comprises:
10%-30% by weight of an agent for skin or mucosa diagnosis or therapy selected from 5-aminolevulinic acid (abbreviated to "ALA") or the esterified derivatives thereof;
0.5%-2% by weight of a mucoadhesive polymer selected from the group consisting of carboxylvinylpolyester (i.e. carbopol or carbomer) and celluloses;
15%-40% by weight of a thermoresponsive polymer having two critical points, which is in solution state at a lower temperature, becomes gel state after the first critical point, and becomes solution state again at a temperature higher than the second critical point, the first critical point is between 25°C and 37°C, and the second critical point is between 45°C and 55°C;
the balance is water and/or a pharmaceutically acceptable excipient, wherein the pH value of the composition is 2-4.

2. The composition according to claim 1, which comprises:
10%-30% by weight of an agent for skin or mucosa diagnosis or therapy, selected from 5-aminolevulinic acid (abbreviated to "ALA") or the esterified derivatives thereof;
1 %-1.5% by weight of a mucoadhesive polymer selected from the group consisting of carbopol 941, carbopol 971P and HPMC;
20%-30% by weight of a thermoresponsive polymer selected from the group consisting of Poloxamer PF127 and poly(N-isopropylacrylamide)(abbreviated to PNIPAAM);
the balance is water and/or a pharmaceutically acceptable excipient, wherein the pH value of the composition is 2-4.

3. The composition according to claim 1, which is used in combination with a light source from a continuous broad band ray, LED or laser to proceed a diagnosis or therapy.

4. The composition according to claim 2, which is used in combination with a light source from a continuous broad band ray, LED or laser to proceeds a diagnosis or therapy.

## Patentansprüche

1. Zusammensetzung, die bei der fotodynamischen Diagnose oder Therapie nützlich ist, welche folgendes umfaßt:
10%-30% (Gew.-%) eines Mittels zur Haut- oder Mukosa-Diagnose oder -Therapie, ausgewählt aus 5-Aminolävulinsäure (abgekürzt mit "ALA") oder den veresterten Derivaten davon;
0,5%-2% (Gew.-%) eines mucoadhäsiven Polymers, ausgewählt aus der Gruppe, bestehend aus Carboxyvinylpolyester (d. h. Carbopol oder Carbomer) und Cellulosen;
15%-40% (Gew.-%) eines thermoresponsiven Polymers mit zwei kritischen Punkten, das sich bei einer niedrigeren Temperatur im Lösungszustand befindet, nach dem ersten kritischen Punkt in den Gelzustand übergeht und bei einer Temperatur, die höher ist als der zweite kritische Punkt wieder in den Lösungszustand übergeht, wobei der erste kritische Punkt zwischen 25°C und 37°C liegt, und der zweite kritische Punkt zwischen 45°C und 55°C liegt;
die Grundlage ist Wasser und/oder ein pharmazeutisch annehmbarer Hilfsstoff, wobei der pH-Wert der Zusammensetzung 2-4 beträgt.

2. Zusammensetzung nach Anspruch 1, die folgendes umfaßt:
10-30% (Gew.-%) eines Mittels zur Haut- oder Mucosa-Diagnose oder -Therapie, ausgewählt aus 5-Aminolävulinsäure (abgekürzt mit "ALA") oder den veresterten Derivaten davon;
1%-1,5% (Gew.-%) eines mucoadhäsiven Polymers, ausgewählt aus der Gruppe, bestehend aus Carbopol 941, Carbopol 971P und HPMC;
20%-30% (Gew.-%) eines thermoresponsiven Polymers, ausgewählt aus der Gruppe, bestehend aus Poloxamer PF 127 und Poly(N-isopropylacrylamid) (abgekürzt mit PNIPAAM);
die Grundlage ist Wasser und/oder ein pharmazeutisch annehmbarer Hilfsstoff, wobei der pH-Wert der Zusammensetzung 2-4 beträgt.

3. Zusammensetzung nach Anspruch 1, welche in Kombination mit einer Lichtquelle mit einer kontinuierlichen breitbandigen Strahlung, einer LED oder einem Laser verwendet wird, um eine Diagnose oder Therapie durchzuführen.

4. Zusammensetzung nach Anspruch 2, welche in Kombination mit einer Lichtquelle mit einer kontinuierlichen breitbandigen Strahlung, einer LED oder einem Laser verwendet wird, um eine Diagnose oder Therapie durchzuführen.

## Revendications

1. Une composition utilisée en diacnostic ou thérapie photodynamique comprenant:
10% à 30% par poids d'un agent pour la diagnostic ou thérapie entanée ou muqueuse choisi à partir d'un acide 5-aminolevulinic (abrégé "ALA") ou de ses derives esterifies;
0,5% à 2% par poids d'un polymère mucoadhésive choisi à partir du groupe composé de carboxylvinylpolyester (c'est-à-dire carbopol ou carbomer) et celluloses;
15% à 40% par poids d'un polymère thermoresponsive comportant deux points critiques, qui est à l'état soluble à basse température, se gélifie après le premier point critique, et revient en état de solution à une température supérieure au second point critique, le premier point critique étant situé entre 25°C et 37°C, le second entre 45°C et 55°C ;
l'équilibre est l'eau et/ou excipient pharmaceutiquement convenable où la valeur de la composition du pH est de 2 à 4.

2. La composition selon la revendication 1 consistant:
10% à 30% par poids d'un agent pour la diagnostic ou thérapie entanée ou muqueuse choisi à partir d'un acide 5-aminolevulinic (abrégé "ALA") ou de ses derives esterifies;
1% à 1,5% par poids d'un polymère mucoadhesive selectioné à partir d'un groupe constitué de carbopol 941, carbopol 971 P et HPMC;
20% à 30% par poids d' un polymère thermoresponsive selectionné à partir d'un groupe constitué de Poloxamer PF127 et poly(N-isoproylacrylamide) (abrégé PNIPAAM);
l'équilibre est l'eau et/ou un dépositaire de médicament pharmaceutique convenable où la valeur de la composition du pH est de 2 à 4.

3. La composition selon la revendication 1, utilisée en combinaison avec une source lumineuse venant d'un large rayon continu, LED ou laser pour procéder à une diagnostic ou une thérapie.

4. La composition selon la revendication numéro 2, utilisée en combinaison avec une source lumineuse venant d'un large rayon continu, LED ou laser pour procéder à un diagnostic ou une thérapie.
